# EUROPEAN PATENT APPLICATION

(11) **EP 3 702 431 A1**
(43) Date of publication of application: **02.09.2020**
(21) Application number: 18869871.6
(22) Date of filing: 26.10.2018
(51) Int. Cl.: C09K 5/14, F25D 9/00

(54) **METHOD AND DEVICE FOR REFRIGERATION INDUCED BY AN EXTERNAL STIMULUS ON A HYBRID ORGANIC-INORGANIC CALORIC MATERIAL**

(30) Priority: 27.10.2017 ES 201731260
(71) Applicant: Universidade da Coruña, 15071 A Coruña (ES)
(72) Inventor: BERMÚDEZ GARCÍA, Juan Manuel, 15071 A Coruña (ES); SEÑARÍS RODRÍGUEZ, María Antonia, 15071 A Coruña (ES); SÁNCHEZ ANDÚJAR, Manuel, 15071 A Coruña (ES); CASTRO GARCÍA, Socorro, 15071 A Coruña (ES); GARCÍA FERNÁNDEZ, Alberto, 15071 A Coruña (ES); ARTIAGA DÍAZ, Ramón Pedro, 15403 Ferrol (ES); LÓPEZ BECEIRO, Jorge José, 15403 Ferrol (ES)
(74) Representative: ZBM Patents - Zea, Barlocci & Markvardsen
(86) International application number: PCT/ES2018/070698
(87) International publication number: WO 2019/081799

(57) **Abstract**

Process of refrigeration induced by an external stimulus comprising the application of an external stimulus selected among hydrostatic pressure, uniaxial pressure, electric field and illumination with light, to an organic-inorganic hybrid material of crystalline structure with hexagonal packing, of formula ABX₃ (I), where A is a given monovalent organic cation or a given mixture of monovalent organic cations or a given mixture of monovalent organic cations and monovalent inorganic cations, B is a given divalent metal cation, a given mixture of divalent metal cations, or a given 50/50% atomic mixture of a monovalent cation and a trivalent cation, and X is a halide anion or a mixture thereof. A device with cooling capacity induced by an external stimulus, comprising the above organic-inorganic hybrid material.

## Description

This application claims the priority of patent application P201731260 filed on October 27, 2017.

### FIELD OF THE INVENTION

The present invention relates to a refrigeration system based on the use of a caloric organic-inorganic hybrid material that is sensitive to an external stimulus, with a device comprising said caloric materials and means for applying said stimulus, as well as with new caloric organic-inorganic hybrid materials.

### STATE OF THE ART

Currently, more than 20% of the world's energy consumption is dedicated to the refrigeration of food, beverages, medicines, electronic devices, machines, vehicles and / or homes. Conventional refrigeration technologies are based on compression / expansion of gases at relatively low pressures, P < 70 bar. However, most of these machines work with toxic and / or polluting refrigerant gases, hazardous chemical compounds (ammonia, NH₃), or greenhouse gases, such as hydrochlorofluorocarbons (HCFCs) and hydrofluorocarbons (HFCs), for example R -134a (CH₂FCF₃) which has a GWP global warming index of 1300 with 1 being CO₂. So that any leakage, breakage or improper waste management at the end of the machine's life is a danger to the environment.

In this context, the European Union will restrict in 2020 (EU Regulation No. 517/2014) the use of greenhouse gases that contribute to global warming, HCFCs and HFCs among others, although it is possible to say that today there is no alternative for replace them that is viable and ecological.

A promising alternative to refrigeration gases are the so-called caloric materials. Caloric materials are solid substances that undergo large thermal changes (isothermal changes in entropy or adiabatic temperature changes) through the application of external stimuli. The stimuli that induce these caloric effects include hydrostatic pressure, uniaxial pressure, magnetic field, or electric field. Thus, known caloric materials are divided into: (i) barocaloric materials (when the caloric effect is induced by a hydrostatic pressure), (ii) elastocaloric materials (when the caloric effect is induced by a uniaxial pressure), (iii) materials magnetocaloric (when the caloric effect is induced by a magnetic field) and (iv) electrocaloric materials (when the caloric effect is induced by an electric field).

The first solid state refrigerator at room temperature was proposed by Gerald Brown in J. Appl. Phys., 1976, vol. 47, pp. 3673-3680 taking advantage of the magnetocaloric effect of gadolinium. Currently, solid caloric materials for refrigeration are based on expensive metals or alloys, usually of rare earths, mainly with magnetocaloric effects. In addition, known caloric materials have a number of drawbacks that hinder their implementation in commercial technologies. In particular, magnetocaloric materials, in addition to being mostly composed of expensive metals or rare earth alloys, need relatively high magnetic fields (larger than 2 T). As for electrocaloric materials, these caloric effects induced by electric field have been observed in thin films with a very small thermal mass, since known electrocaloric materials tend to rapidly degrade in working conditions. On the other hand, baro- and elastocaloric materials have the advantage that the hydrostatic and uniaxial pressure are simple to apply and do not lead to the rapid degradation of the material under working conditions. However, very few materials with baro- and elastocaloric effect are known today, which are also generally expensive materials and show a limited response to pressure and, therefore, require relatively high pressures (P > 1000 bar). (cf. X. Moya et al., Nature Materials, 2014, vol.13, pp. 439-450).

Organic-inorganic hybrid materials have undergone rapid development in recent years. These organic-inorganic hybrid materials integrate inorganic cations linked by organic or inorganic anions and form a mono, bi- or three-dimensional network that can host organic species (both molecular, cationic or anionic) and sometimes mixtures of organic species with inorganic cations.

An organic-inorganic hybrid material with barocaloric effect is known for refrigeration applications, in particular, the compound [(CH₃CH₂CH₂)₄N] [Mn(N(CN)₂)₃] with cubic packing crystalline structure based on [Mn((N(CN)₂)₃)₆] octahedra that share their vertices forming cavities where the [(CH₃CH₂CH₂)₄N]+ cations are located (cf. JM Bermúdez-García et al. Nature Communications, 2017, 8, 15715). It has also been theoretically predicted other 16 organic-inorganic hybrid materials could show barocaloric effect, however this effect has not been experimentally demonstrated yet. (cf. J. M. Bermúdez-García et al. in J. Phys. Chem. Lett., 2017, vol. 8, pp. 4419-4423). In particular, among the mentioned materials is the family of [CH₃NH₃]PbX₃ (X = I-, CI-, Br-) with cubic packing crystalline structure based on [PbX₆] octahedra that share their vertices forming cavities where the [CH3NH3]+ cations are located. Nevertheless, this family has been predicted to display a relatively bad barocaloric effect for refrigeration applications due to a low sensitivity to pressure and/or a very low phase transition temperature (parameter that may indicate the working temperature of such materials). Therefore, the fact that the caloric effect occurs at very low temperatures would hinder any possible practical application as a cooling material.

In view of the state of the art, there is still a need to find new solid caloric materials useful in refrigeration applications that are more efficient and give rise to economical and environmentally friendly technological solutions.

### SUMMARY OF THE INVENTION

The inventors have noticed a new family of organic-inorganic hybrid materials with general formula ABX₃ (I) with an improved barocaloric effect that can be led to more efficient refrigeration applications. In these materials, A are medium-sized organic cations (those cations with a volume between 13 Å³ and 275 Å³) or mixtures of these organic cations with inorganic cations, B are metal cations and X are halides, they exhibit hexagonal packing crystalline structure based on [BX₆] octahedra sharing vertices and faces, and forming cavities where A cations are located, such materials can undergo a solid-solid phase transition induced by an external stimulus such as hydrostatic pressure, which results in an improved barocaloric effect that can be used more efficiently for cooling applications. The cooling capacity of these materials is associated with isothermal entropy changes or adiabatic temperature changes induced by external stimuli in the vicinity of the solid-solid transition temperature, which is what sets the working temperature of such materials, that is, the temperature at which the material itself can work in a refrigeration device.

An advantage of using organic-inorganic hybrid materials such as those of the present invention in a refrigeration process is that they are solid materials, which avoids the use of hazardous gases and pollutants, allows a compact cooling system, are easy of handling and in the case of an accidental leak they are easier to contain than a gas or a liquid. The organic-inorganic hybrid materials of the present invention are much cheaper materials than the rare earth metals and alloys that are being used as caloric materials, are sensitive to hydrostatic pressure and uniaxial pressure, and integrate elements sensitive to the magnetic field, electric field and the light, being able to induce a caloric effect by one or several of these stimuli. Additionally, these materials are lightweight, which lightens the weight of the cooling device. They are also more flexible than the known caloric materials based on the rare earth metals and alloys that are being used, being able to include flexible devices such as shoe insoles, textiles, the new generation of flexible smartphones, etc.

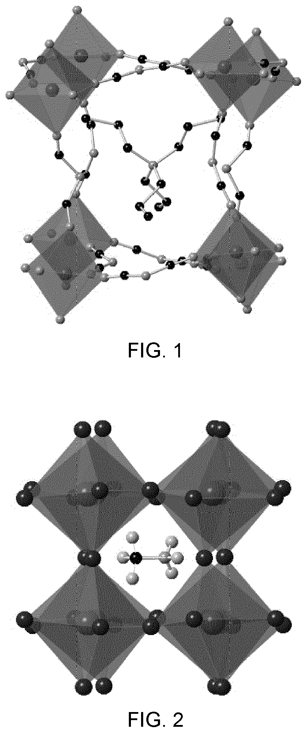

Unlike [(CH₃CH₂CH₂)₄N][Mn(N(CN)₂)₃], which has a cubic packing crystalline structure, or the family of [CH₃NH₃]PbX₃ (X = I-, CI-, Br-) materials, which also have cubic packing crystalline structure, the organic-inorganic hybrid materials with general formula ABX₃ of the present invention have a hexagonal packing crystalline structure, which allows for a much higher pressure sensitivity of these compounds with respect to the materials with cubic packing crystalline structure (cf. FIGs 1-3).

The barocaloric effect of the compounds of the present invention is considerably larger than that predicted for the [CH₃NH₃]PbX₃ (X = I-, CI-, Br-) compounds, which are the chemically closest compounds, and also have the additional advantage of that have a better working temperature, much closer to room temperature, which greatly facilitates its application as a cooling materials.

The compounds of the present invention have a barocaloric effect similar to the [(CH₃CH₂CH₂)₄N][Mn(N(CN)₂)₃] compound although they have additional advantages since they can be manufactured through a more simple, ecological and economical method than the [(CH₃CH₂CH₂)₄N][Mn(N(CN)₂)₃] material.

For the purposes of the invention, not only hydrostatic pressure can be used as a stimulus but also other stimuli that give rise to the solid-solid phase transition such as uniaxial pressure, electric field or light illumination. These effects are derived according to theoretical calculations obtained from the structural data of the compounds of the present invention. According to the inventors' knowledge, no organic-inorganic hybrid material with elastocaloric, electrocaloric, or photocaloric effect has been described for refrigeration applications.

The barocaloric effect and the elastocaloric effect are both induced by pressure, the first by hydrostatic pressure (pressure exerted equally on the entire surface of the material) and the second by uniaxial pressure (pressure exerted only along one axis). Large thermal changes (isothermal entropy changes or adiabatic temperature changes) occur through the application of a hydrostatic or uniaxial pressure. The elastocaloric effect is advantageous in those cases in which the material deforms more on one axis than on another, but in principle both effects can be considered similar. In the case of the photocaloric effect and the electrocaloric effect, large thermal changes (isothermal entropy changes or adiabatic temperature changes) also occur through the application of illumination (light, laser, etc.) or the application of an electric field respectively.

Finally, the compounds of the present invention have the additional advantage over other known organic-inorganic hybrid materials, which is that they are thermally stable and/or also under light conditions even under humidity conditions of up to 65% for a prolonged period of time.

Therefore, a first aspect of the present invention relates to a refrigeration process induced by an external stimulus which comprises applying an external stimulus selected from hydrostatic pressure, uniaxial pressure, electric field and illumination with light, to an organic-inorganic hybrid material of hexagonal packing crystalline structure of general formula ABX₃ (I), where: A is selected from the group consisting of a monovalent organic cation, a mixture of monovalent organic cations, and a mixture of monovalent organic cations and monovalent inorganic cations; the monovalent organic cation is selected from the group consisting of hydrazinium ([NH₃NH₂]⁺), hydroxylammonium ([NH₃OH]⁺), formamidinium ([CH(NH₂)₂]⁺), guanidinium ([C(NH₂)₃]⁺ ), azetidinium ([C₃NH₈]⁺), dimethylammonium ([(CH)₂NH₂]⁺), ethylammonium ([CH₃CH₂NH₃]⁺), azetamidinium ([CH₃C(NH₂)₂]⁺), tetramethylammonium ([(CH₃)₄N]⁺), imidazolium ([C₃N₂H₅]⁺), trimethylammonium ([(CH₃)₃NH]⁺), isopropylammonium ([(CH₃)₂CNH₃]⁺), pyrrolidinium ([(C₄H₄)NH₂]⁺), isobutylammonium ([(CH₃)₂CH₃)₂N]⁺), diethylammonium ([(CH₃CH₂)2NH₂]⁺), and phenylammonium ([(C₆H₅)NH₃]⁺); the monovalent organic cation mixture is a mixture of any of the aforementioned organic cations, also including [CH₃NH₃]⁺; and the mixture of organic cations and monovalent inorganic cations is a mixture of any of the aforementioned organic cations with one or more inorganic cations selected from the group consisting of Cs⁺ , Rb⁺ , NH₄⁺ ; B is selected from the group consisting of: a divalent metal cation, a mixture of divalent metal cations, and a 50/50% atomic mixture of a monovalent cation and a trivalent cation, where: the divalent metal cation is Select from the group consisting of: Mg²⁺ , Ca²⁺, Sr²⁺, Mn²⁺, Fe²⁺, Co²⁺, Ni²⁺, Cu²⁺, Zn²⁺, Cd²⁺, Pb²⁺, Sn²⁺, and Sb²⁺; the monovalent metal cation is selected from the group consisting of: Ag⁺, Na⁺, K⁺, Tl⁺, and Cu⁺; and the trivalent cation is selected from the group consisting of: Cr³⁺, Fe³⁺, Bi³⁺, In³⁺; Y³⁺, Lu³⁺, La³⁺, Ce³⁺, Pr³⁺, Nd³⁺, Pm³⁺, Sm³⁺, Eu³⁺, Gd³⁺, Tb³⁺, Dy³⁺, Ho³⁺, Er³⁺, Tm³⁺, and Yb³⁺; and X is a halide anion selected from the group consisting of F⁻, Cl⁻, Br, I⁻, and a mixture thereof.

A "monovalent organic cation" may be an organic cation of charge +1. The monovalent organic cation A of the present invention has an average size with a volume of between 13 Å³ to 275 Å³. A "divalent organic cation" may be an organic cation with charge 2+. A "trivalent organic cation" may be an organic cation with charge 3+.

In a particular embodiment, the refrigeration process is one where in the organic-inorganic hybrid material, A is a monovalent organic cation that is selected from the group consisting of dimethylammonium ([(CH)2NH₂]⁺), ethylammonium ([CH₃CH₂NH₃]⁺), tetramethylammonium ([(CH₃)₄N]⁺), trimethylammonium ([(CH₃)₃NH]⁺), isopropylammonium ([(CH₃)₂CNH₃]⁺), isobutylammonium ([(CH₃)₂CH₃)₂N]⁺), and diethylammonium ([(CH₃CH₂)₂NH₂]⁺).

In another particular embodiment, the refrigeration process is one where in the organic-inorganic hybrid material A is [(CH₃)₂NH₂]⁺.

In another particular embodiment, the refrigeration process is one where in the organic-inorganic hybrid material, A is a monovalent organic cation that is selected from the group consisting of hydrazinium ([NH₃NH₂]⁺), hydroxylammonium ([NH₃OH]⁺), formamidinium ([CH(NH₂)₂]⁺), guanidinium ([C(NH₂)₃]⁺).

In another particular embodiment, the refrigeration process comprises a organic-inorganic hybrid material where A is a monovalent organic cation that is selected from the group consisting of, imidazolium ([C₃N₂H₅]⁺), pyrrolidinium ([(C₄H₄)NH₂]⁺), and phenylammonium ([(C₆H₅)NH₃]⁺).

In another particular embodiment, the refrigeration process comprises an organic-inorganic hybrid material where A is a mixture of monovalent organic cations including [CH₃NH₃]⁺ as defined above. In another particular embodiment, the cooling process comprises an organic-inorganic hybrid material where A is a 60/40% atomic mixture of [(CH₃)₂NH₂]⁺/[(CH₃)₂CH₃)₂N]⁺.

In another particular embodiment, the refrigeration process comprises an organic-inorganic hybrid material where A is a mixture of monovalent organic cations and monovalent inorganic cations. In another particular embodiment, the refrigeration process comprises an organic-inorganic hybrid material where A is a mixture of [(CH₃)₂NH₂]⁺/ Cs⁺.

In another particular embodiment the refrigeration process comprises an organic-inorganic hybrid material where A is a 60/40% atomic mixture of [(CH₃)₂NH₂]⁺/ [(CH₃)₂CH₃)₂N]⁺, or a 75/25% atomic mixture of [(CH₃)₂NH₂]⁺/ Cs⁺.

In a particular embodiment, the refrigeration process is one where the trivalent cation in B is selected from the group consisting of: Cr³⁺, Fe³⁺, Bi³⁺, In³⁺, and Y³⁺.

In a particular embodiment, the refrigeration process is one where the trivalent cation in B is selected from the group consisting of: Lu³⁺, La³⁺, Ce³⁺, Pr³⁺, Nd³⁺, Pm³⁺, Sm³⁺, Eu³⁺, Gd³⁺, Tb³⁺, Dy³⁺, Ho³⁺, Er³⁺, Tm³⁺, and Yb³⁺.

In another particular embodiment, the refrigeration process comprises a organic-inorganic hybrid material where B is a divalent metal cation.

In another particular embodiment, the refrigeration process comprises a organic-inorganic hybrid material where B is a divalent metal cation that is selected from the group consisting of: Pb²⁺, Mn²⁺.

In another particular embodiment, the refrigeration process comprises a organic-inorganic hybrid material where B is a mixture of divalent metal cations or a 50/50% atomic mixture of a monovalent cation and a trivalent cation.

In another particular embodiment, the refrigeration process comprises a organic-inorganic hybrid material where B is selected from the group consisting of: a mixture of Mn²⁺/Co²⁺, and a mixture of Ag⁺/Bi³⁺.

In another particular embodiment, the cooling process comprises a organic-inorganic hybrid material where B is selected from the group consisting of: Pb²⁺, Mn²⁺, a 60/40% atomic mixture of Mn²⁺/Co²⁺, and a 50/50% atomic mixture of Ag⁺/Bi³⁺.

In another particular embodiment, the refrigeration process comprises a organic-inorganic hybrid material where X is Cl⁻.

In another particular embodiment, the refrigeration process comprises a organic-inorganic hybrid material where X is a mixture of Cl⁻/I⁻, or a mixture of I⁻/ Cl⁻/Br⁻.

In another particular embodiment, the refrigeration process comprises a organic-inorganic hybrid material where X is a 60/40% atomic mixture of Cl⁻/I⁻, or a 30/50/20% atomic mixture of I⁻/Cl⁻/Br⁻.

Unless otherwise indicated, all percentages mentioned in this document with respect to the elements of the organic-inorganic hybrid material are expressed as an atomic percentage. The atomic percentage means the number of atoms of an element in 100 representative atoms of a substance.

In a preferred embodiment, the refrigeration process is one where the organic-inorganic hybrid material is [(CH₃)2NH₂]PbCl₃.

The cooling process is carried out inside a device by the cyclical and repetitive application of an external stimulus on the organic-inorganic hybrid material, the process that generally comprises the following steps: a) Applying an external stimulus with which the material is heats up; b) Maintaining the stimulus for a certain period of time, which gives off heat that is directed outside the device; c) Removing the stimulus so that the material cools; and d) Using the cooled material to absorb heat from inside the device to be cooled.

As an example, in the first step an external stimulus such as hydrostatic pressure is applied causing the organic-inorganic material to heat up. In the second step, the applied external stimulus remains constant for a time so that the heat generated is directed towards a heat sink, for example, this may be a heat sink. This heat can be directed to the heat sink either by direct contact of the material with this sink or by a heat transfer fluid such as air, water, alcohols, etc. In the third step, once the organic-inorganic hybrid material has released that heat, the applied stimulus is removed which produces a cooling of the organic-inorganic hybrid material. In the fourth step, keeping the stimulus removed for a certain time, the organic-inorganic hybrid material (that has been cooled in the previous step) absorbs the heat of the reservoir to be cooled, for example the inside of a refrigerator. This heat from inside the reservoir is absorbed either by direct contact of the material with this reservoir or by a heat transfer fluid such as air, water, alcohols, etc. This four-steps process is repeated cyclically in a specific and predetermined time in which the stimulus actuator, for example a piston, exerts and removes the pressure automatically and infinitely.

In a preferred embodiment, the cooling process is one where the external stimulus is hydrostatic or uniaxial pressure. In a particular embodiment, the applied hydrostatic or uniaxial pressure is between 20 and 1000 bar (2-100 MPa). In a more preferred embodiment, the applied hydrostatic/uniaxial pressure is 20-100 bar (2-10 MPa). In a more preferred embodiment, the applied hydrostatic/uniaxial pressure is between 40-70 bar (4-7 MPa). In an even more preferred embodiment, the applied hydrostatic pressure is 69 bar (6.9 MPa).

In a particular embodiment, the refrigeration process is one where the external stimulus is light. In a particular embodiment, the intensity of the applied light is between 0.1-1000 mW cm⁻². In another particular embodiment, the intensity of the light is 1-100 mW cm⁻²: In another particular embodiment, the intensity of the light is approximately 10 mW cm⁻². In another particular embodiment, the wavelength of the light is between 200-1000 nm. In another particular embodiment, the wavelength of the light is approximately 450 nm.

In a particular embodiment, the cooling process is one where the external stimulus is an electric field. In another particular embodiment, the electric field applied in the case of using electrocaloric materials is a voltage between 1 - 500 V.

In another particular embodiment, the electric field applied in the case of using electrocaloric materials is a voltage between 10 - 100 V. In another particular embodiment, the applied electric field is a voltage of approximately 40 V.

Generally, the time that the applied stimulus is maintained in the case of barocaloric or elastocaloric materials as well as photocaloric or electrocaloric materials is between 0.05-60 seconds. Preferably, the time that the applied stimulus is maintained is between 1-10 seconds. More preferably, the time that the applied stimulus is maintained is approximately 1 second.

Generally, the time that the stimulus is maintained withdrawn in the case of barocaloric or elastocaloric materials as well as photocaloric or electrocaloric materials is between 0.05 - 60 seconds. Preferably, the time that the applied stimulus is maintained withdrawn is between 1-10 seconds. More preferably, the time that the removed stimulus is maintained withdrawn is approximately 1 second.

The term "approximately" in this document means the value indicated in the corresponding unit ± 5%.

The organic-inorganic hybrid materials used in the refrigeration process of the present invention allow to work in a temperature range between 0 ° C and 120 ° C. Preferably, the working temperature range is between 7 ° C and 70 ° C. More preferably, the working temperature range is between 40-65 ° C.

In a particular embodiment of the cooling process, the organic-inorganic hybrid material is [(CH₃)₂NH₂]PbCl₃ and the working temperature is between 42 and 65 °C, preferably between 42 and 54 °C.

Also part of the invention is the use of the organic-inorganic hybrid material of hexagonal packing crystalline structure of general formula ABX₃ (I), as defined above, as caloric material for cooling devices. Particular and preferred embodiments of the refrigeration process described above in relation to the materials are also particular and preferred embodiments for this aspect of the invention.

These materials can be incorporated in a cooling device. The device can be used in different sectors such as the air cooling sector conditioning, refrigerators, refrigeration of electronic devices, cars, textiles, clothes, shoes, etc.

Another aspect of the present invention is related to a device with cooling capacity induced by an external stimulus, comprising: (1) An organic-inorganic hybrid material of general formula ABX₃ (I) and hexagonal packing crystalline structure such as has been defined above; (2) means to cyclically exert the stimulus for a certain period of time on the organic-inorganic hybrid material and then remove it, where the external stimulus is selected from the group consisting of hydrostatic pressure, uniaxial pressure, electric field and light illumination.

In a particular embodiment, the device of the invention is one that has means for applying a hydrostatic pressure as an external stimulus. In another particular embodiment, the device of the invention is one that has means for applying a uniaxial pressure, an electric field or illumination with light. The particular/preferred values of hydrostatic pressure, uniaxial pressure, electric field or illumination indicated above for the cooling process are also particular/preferred values for the device.

For example, the means for cyclically exerting and removing an external stimulus selected from hydrostatic pressure, uniaxial pressure, electric field and illumination with light can be the following: for pressure: a piston, a fluid or a person hand or finger on a screen (or any actuator that exerts hydrostatic or uniaxial pressure); for the electric field: an electric circuit that transmits electric current to the material; and for the light: a bulb, lamp, led, laser, flashlight, etc.

In a particular embodiment, the device of the invention further comprises: (3) a heat sink that is responsible for removing heat to the outside; (4) optionally a heat exchanger fluid; and (5) a reservoir or enclosure that needs to be cooled. The sink could be for example a fan, a heat sink, etc. The heat exchanger fluid is optional and if present it could be selected from air, water, alcohols, etc. For example, in the case of a fridge, the reservoir or enclosure that needs to be cooled would be the inside of the fridge. For further example, in the case of a smartphone, the reservoir would be the inside of the smartphone. For further example, in the case of shoes, the reservoir would be the inside of the shoes.

In addition to this cooling device, the hybrid material may be contained in form of a powder, suspended in a fluid (or in a solid matrix) that acts as a heat exchanger and/or improves thermal conductivity or as a thin film (thin layer), for example on screens for electronic devices. In a particular embodiment of the device, the organic-inorganic hybrid material of general formula ABX₃ is in powder form, suspended in a heat exchanger fluid such as an oil, silicone, water, alcohols, etc., suspended in a heat conductive solid matrix (for example a composite of the caloric hybrid material and a polymer, metallic alloy, or steel), or in the form of a thin film on top of an electrically conductive substrate (for example a coated glass of tin oxide with fluoride and/or with indium).

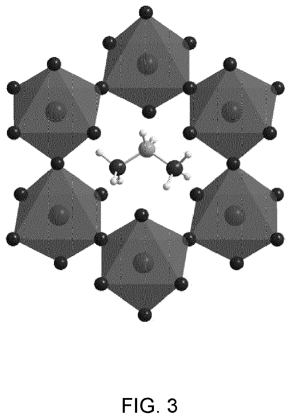

The organic-inorganic hybrid material of formula [(CH₃)₂NH₂]PbCl₃ (Ia) (i.e. the compound of formula ABX₃ (I) where A is dimethylammonium ([(CH)₂NH₂]⁺), B is Pb²⁺ and X is Cl⁻) exhibits the crystal structure illustrated in FIG. 3 as obtained by single-crystal X-ray diffraction.

To obtain the single-crystal X-ray crystal structure of the organic-inorganic hybrid material of formula [(CH₃)₂NH₂]PbCl₃ (la), a suitable single-crystal was selected from the sample of [(CH₃)₂NH₂]PbCl₃ to collect diffraction data at Ambient temperature in a Bruker Kappa diffractometer equipped with an APEX II CCD detector and using MoKa monochromatic radiation (0.71073 Å). The crystal was mounted on a MiTeGen MicroMountTM using Paratone® (Chevron Corporation). To carry out this experiment, the crystal was measured at room temperature. Data collection, integration and reduction were carried out with the software package APEX2 V2015.9-0 (Bruker AXS, 2015), which includes the programs detailed below. Intensity integration was performed with SAINT 8.34A and corrected for Lorentz and polarization effects and also for absorption using multiple scanning methods based on equivalent symmetry data using SADABS 2008/1 or TWINABS 2012/1 depending on the presence of twinnings. A unique network was found for the crystal collected at room temperature (T = 20-25° C).

The structure was solved by the direct method using the SHELXT2014 program and refined by the least squares method in SHELXL2014 / 7. The presence of twinnings was clear from the visual inspection of collected diffraction images. The data set was indexed using CELL_NOW 2008/4 obtaining three orientation matrices that interpret all diffraction maxima. The integration of the reflections was carried out taking into account the orientation matrices of the three domains mapped simultaneously. To refine the structure, anisotropic thermal factors were used for the non-H atoms. For the hydrogen atoms of the cation [(CH₃)₂NH₂]⁺ they could not be found on the Fourier map due to the disorderly arrangement of this cation. All hydrogen atoms were refined using the conduction model implemented in SHELXL2014 / 7.

Single crystal X-ray diffraction at room temperature reveals that the [(CH₃)₂NH₂]PbCl₃ compound has a hexagonal packing crystalline structure and shows the bond distances indicated in Table 1.

**Table 1: selected atomic distances for the[(CH₃)₂NH₂]PbCl₃ compound at room temperature.**

| | |
|---|---|
| Pb1-Cl1 | 2.698(7) |
| Pb1-Cl2 | 2.738(7) |
| Pb1-Cl3 | 3.044(6) |
| Pb1-Cl4 | 2.773(6) |
| Pb1-Cl5 | 3.077(7) |
| Pb1-Cl6 | 3.092(7) |
| Pb2-Cl7 | 2.705(7) |
| Pb2-Cl8 | 2.761(8) |
| Pb2-Cl9 | 2.883(7) |
| Pb2-Cl10 | 2.956(7) |
| Pb2-Cl11 | 2.985(6) |
| Pb2-Cl12 | 3.112(6) |
| C1-N1 | 1.472(6) |
| C2-N1 | 1.468(8) |
| C3-N2 | 1.482(7) |
| C4-N2 | 1.477(8) |

The crystal structures with cubic packing of the organic-inorganic hybrid materials of the comparative compounds [(CH₃CH₂CH₂)₄N][Mn(N(CN)₂)₃] and [CH₃NH₃]PbCl₃ can also be elucidated by single-crystal X-ray diffraction carried out in a similar way than in the case of the hybrid material [(CH₃)₂NH₂]PbCl₃.

The compound [(CH₃)₂NH₂]PbCl₃ is thermally stable up to 190 °C. The stability of this compound is similar to that predicted for the comparative compounds of formula [CH₃NH₃]PbX₃. Advantageously, the compound [(CH₃)₂NH₂]PbCl₃ remains stable under conditions of ambient temperature and illumination, and under humidity conditions of up to 65%, for at least one year. Meanwhile the comparative compounds [CH₃NH₃]PbX₃ in less than 24 hours absorb water that modifies its properties and structure.

The organic-inorganic hybrid material of formula [(CH₃)₂NH₂]PbCl₃ can be obtained by a process comprising a solid state reaction activated by mechanical means using [(CH₃)₂NH₂]Cl and PbCl₂. The process is simple and uses mild conditions. It does not need a solvent and is therefore ecological.

In a particular embodiment, the above reaction is carried out at room temperature. Here, ambient temperature refers to a temperature between 20 and 25 ° C. In another particular embodiment, the mechanical means are means for carrying out a grinding. In another particular embodiment, the mechanical mixing is carried out with equimolar amounts of the starting compounds.

The organic-inorganic hybrid materials used in the process and device of the present invention can be obtained similarly to the organic-inorganic hybrid material of formula [(CH₃)₂NH₂]PbCl₃ from the corresponding organic cation halides and metal cation halides. Other synthetic methods are reported in the literature, such as those described in M. G. Kanatzidis et al. (Inorganic Chemistry, 2017, vol. 56, pp. 56-73) and the articles referenced there. A skilled person in the art would know how to adapt the methods of these documents for the preparation of organic-inorganic hybrid materials.

Throughout the description and the claims the word "comprises" and its variants are not intended to exclude other technical characteristics, additives, components or steps. For those skilled in the art, other objects, advantages and features of the invention will be derived partly from the description and partly from the practice of the invention. The following examples and figures are provided for illustration, and are not intended to be limiting of the present invention. In addition, the present invention covers all possible combinations of particular and preferred embodiments indicated herein.

### BRIEF DESCRIPTION OF THE FIGURES

FIG. 1: Crystal structure of the comparative compound [(CH₃CH₂CH₂)₄N][Mn(N(CN)₂)₃] obtained by single-crystal X-ray diffraction.
FIG. 2: Crystal structure of the comparative compound [CH₃NH₃]PbCl₃ obtained by single-crystal X-ray diffraction.
FIG. 3: Crystal structure of the compound [(CH₃)₂NH₂]PbCl₃ obtained by single-crystal X-ray diffraction.
FIG. 4: Barocaloric effect of the comparative compound [(CH₃CH₂CH₂)₄N][Mn(N(CN)₂)₃].
FIG. 5: Barocaloric effect of compound [(CH₃)₂NH₂]PbCl₃.

### EXAMPLES

### Example 1: Preparation of [(CH₃)₂NH₂]PbCl₃

The material [(CH₃)₂NH₂]PbCl₃ was obtained by mechanochemical synthesis at room temperature using the starting compounds [(CH₃)₂NH₂]Cl and PbCl₂. Equimolar amounts of [(CH₃)₂NH₂]Cl and PbCl₂ were used for this purpose and ground in a mortar for 15 minutes until a homogeneous powder was visually obtained.

Compound [(CH₃)₂NH₂]Cl was previously synthesized by reaction of equimolar amounts of dimethylamine in aqueous solution (40% weight of (CH₃)₂NH in H₂O) and of hydrochloric acid in aqueous solution (37% weight of HCI in H₂O). This mixture was stirred for 15 minutes in an ice bath. Crystallization of dimethylammonium chloride was carried out by evaporating the solvent in a rotary evaporator until the appearance of a white solid precipitate of microcrystals. This solid was filtered and washed with diethyl ether several times and dried under vacuum overnight.

The PbCl₂ compound was synthesized by reacting equimolar amounts of sodium chloride (or potassium chloride) in saturated aqueous solution and lead nitrate in aqueous solution. This solution mixture was stirred for 15 minutes in an ice bath. The crystallization of PbCl₂ was carried out by evaporating the solvent in a rotary evaporator until the appearance of a white solid precipitate of microcrystals. This solid was filtered and washed with diethyl ether several times and dried under vacuum overnight.

Compounds [(CH₃)₂NH₂]Cl and PbCl₂ can also be obtained commercially.

To obtain single crystals of the compound and to obtain greater purity, this compound was dissolved in an organic solvent (such as N, N-dimethylformamide or dimethyl sulfoxide) in concentrations between 15% - 45% by weight. Subsequently, the solvent was allowed to evaporate at room temperature for one week to obtain single crystals or to purify the compound. The obtained material was characterized by single-crystal X-ray diffraction (see FIG. 3).

### Example 2: Deposition of a [(CH₃)₂NH₂]PbCl₃ thin film on a substrate

To deposit a [(CH₃)₂NH₂]PbCl₃ thin film on a substrate, this compound was dissolved in an organic solvent (either N, N-dimethylformamide or dimethyl sulfoxide) in concentrations between 15% - 45% by weight. Subsequently, the solvent was allowed to evaporate on a substrate by means of rotation at 2000 rpm for 60 seconds to obtain a thin layer of the hybrid compound.

### Example 3: Barocaloric Effect

The differential scanning calorimetry technique was used to study the caloric effect of the material. To do this, samples of about 5 mg of the material are analyzed in a TA Instruments MDSC Q2000 equipped with a pressure cell. The samples were cyclically heated and cooled at speeds between 1 °C min⁻¹ and 20 °C min⁻¹, from room temperature to 150 °C, under nitrogen atmosphere. These heating and cooling cycles were performed at different nitrogen pressures from 1 bar to 69 bar, with a constant flow of nitrogen of 50 ml min⁻¹. The pressure cell was calibrated for each of these pressures using an indium standard. The barocaloric effect was obtained as the difference of isobaric entropy change at the pressure of 69 bar and change of isobaric entropy at the pressure of 1 bar, in units of J kg⁻¹ K⁻¹.

| Material | Barocaloric Effect (J kg⁻¹ K⁻¹) | Transition temperature (°C) | Pressure sensitivity (K kbar⁻¹) |
|---|---|---|---|
| Comparative example 1 [(CH₃CH₂CH₂)₄N][Mn(N(CN)₂)₃] | 37.0 (experimental) | 57 | 23.1 |
| Comparative example 2 [CH₃NH₃]Pbl₃ | 15.65 (theoretical) | 57 | 3.5 |
| Comparative example 3 [CH₃NH₃]PbBr₃ | 23.38 (theoretical) | -124 | 9.6 |
| Comparative example 4 [CH₃NH₃]PbCl₃ | 28.93 (theoretical) | -102 | 5.7 |
| Example 1 [(CH₃)₂NH₂]PbCl₃ | 31.0 (experimental) | 42 | 23.2 |

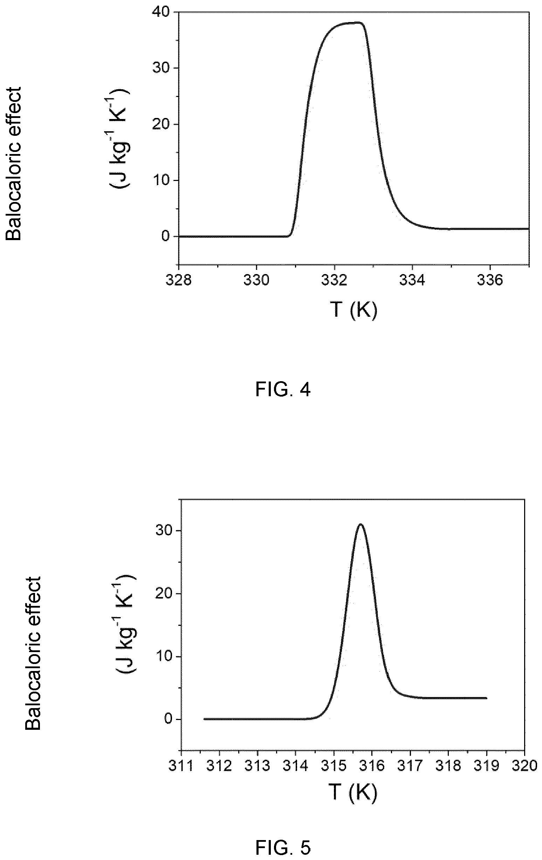

The barocaloric effect of the comparative example 1 and of the example 1 of the invention are illustrated in FIGS. 4 and 5 respectively.

The barocaloric effect of the [(CH₃)₂NH₂]PbCl₃ compound is considerably larger than that predicted for the compounds of comparative examples 2-4 which are the chemically closest compounds. Also, the [(CH₃)₂NH₂]PbCl₃ compound has a better working temperature, much closer to room temperature than that of comparative the compounds, which largely facilitates its application as cooling material.

### REFERENCES

### Non-patent literature

- EU Regulation No. 517/2014
- Gerald Brown in J.Appl. Phys., 1976, vol.47, pp. 3673-3680
- J. M. Bermúdez-García et al., Nature Communications, 2017, 8, 15715
- J. M. Bermúdez-García et al., J. Phys. Chem. Lett., 2017, vol. 8, pp. 4419-4423 - X. Moya et al., Nature Materials, 2014, vol. 13, pp. 439-450
- M. G. Kanatzidis et al., Inorganic Chemistry, 2017, vol. 56, pp. 56-73

## Claims

1. A refrigeration process which comprises applying an external stimulus selected from hydrostatic pressure, uniaxial pressure, electric field, and illumination with light to an organic-inorganic hybrid material of hexagonal packing crystal structure of general formula:
ABX₃ (I)
where:
A is selected from the group consisting of a monovalent organic cation, a mixture of monovalent organic cations, and a mixture of monovalent organic cations and monovalent inorganic cations;
the monovalent organic cation is selected from the group consisting of [NH₃NH₂]⁺ , [NH₃OH]⁺ , [CH(NH₂)₂]⁺ , [C(NH₂)₃]⁺ , [C₃NH₈]⁺ , [(CH₃)₂NH₂]⁺ , [CH₃CH₂NH₃]⁺, [CH₃C(NH₂)₂]⁺ , [(CH₃)₄N]⁺, [C₃N₂H₅]⁺, [(CH₃)₃NH]⁺, [(CH₃)₂CNH₃]⁺, [(C₄H₄)NH₂]⁺, [(CH₃)₂CH₃)₂N]⁺, [(CH₃CH₂)₂NH₂]⁺, and [(C₆H₅)NH₃]⁺ ; and
the monovalent organic cation mixture is a mixture of any of the organic cations mentioned, including [CH₃NH₃]⁺; and
the mixture of organic cations and monovalent inorganic cations is a mixture of any of the aforementioned organic cations with one or more inorganic cations selected from the group consisting of Cs⁺, Rb⁺, NH₄⁺;
B is selected from the group consisting of: a divalent metal cation, a mixture of divalent metal cations, and a 50/50% atomic mixture of a monovalent cation and a trivalent cation, where:
The divalent metal cation is selected from the group consisting of: Mg²⁺, Ca²⁺, Sr²⁺, Mn²⁺, Fe²⁺, Co²⁺, Ni²⁺, Cu²⁺, Zn²⁺, Cd²⁺, Pb²⁺, Sn²⁺, and Sb²⁺;
the monovalent metal cation is selected from the group consisting of: Ag⁺, Na⁺, K⁺, Tl⁺, and Cu⁺;
the trivalent cation is selected from the group consisting of: Cr³⁺, Fe³⁺, Bi³⁺, In³⁺; Y³⁺, Lu³⁺, La³⁺, Ce³⁺, Pr³⁺, Nd³⁺, Pm³⁺, Sm³⁺, Eu³⁺, Gd³⁺, Tb³⁺, Dy³⁺, Ho³⁺, Er³⁺, Tm³⁺ and Yb³⁺;
X is a halide anion selected from the group consisting of F⁻, Cl⁻, Br, I⁻, and a mixture thereof.

2. The refrigeration process according to claim 1, wherein A is a monovalent organic cation selected from the group consisting of: [(CH₃)₂NH₂]⁺, [CH₃CH₂NH₃]⁺, [(CH₃)₄N]⁺, [(CH₃)₃NH]⁺, [(CH₃)₂CNH₃]⁺, [(CH₃)₂CH₃)₂N]⁺, and [(CH₃CH₂)₂NH₂]⁺.

3. The refrigeration process according to claim 2, wherein A is [(CH₃)₂NH₂]⁺.

4. The refrigeration process according to claim 1, wherein A is a 60/40% atomic mixture of [(CH₃)₂NH₂]⁺ / [(CH₃)₂CH₃)₂N]⁺ or a 75/25% atomic mixture of [(CH₃)₂NH₂]⁺ / Cs⁺.

5. The refrigeration process according to any of the claims 1-4, wherein the trivalent cation is selected from the group consisting of: Cr³⁺, Fe³⁺, Bi³⁺, In³⁺, and Y³⁺.

6. The refrigeration process according to any of the claims 1-4, wherein the trivalent cation is selected from the group consisting of: Lu³⁺, La³⁺, Ce³⁺, Pr³⁺, Nd³⁺, Pm³⁺, Sm³⁺, Eu³⁺, Gd³⁺, Tb³⁺, Dy³⁺, Ho³⁺, Er³⁺, Tm³⁺, and Yb³⁺.

7. The refrigeration process according to any of the claims 1-4, wherein B is selected from the group consisting of a divalent metal cation.

8. The refrigeration process according to any of the claims 1-4, wherein B is selected from the group consisting of: Pb²⁺, Mn²⁺, a 60/40% atomic mixture of Mn²⁺ / Co²⁺, and a 50/50% atomic mixture of Ag⁺/ Bi³⁺.

9. The refrigeration process according to any of the claims 1-8, wherein X is Cl⁻.

10. The refrigeration process according to any of the claims 1-8, wherein X is a 60/40% atomic mixture of Cl⁻/ I⁻, or a 30/50/20% atomic mixture of I⁻/ Cl⁻ / Br.

11. The refrigeration process according to claim 1, wherein the organic-organic hybrid material is [(CH₃)₂NH₂]PbCl₃.

12. The refrigeration process according to any of the claims 1-11, wherein the external stimulus is hydrostatic pressure.

13. The refrigeration process according to any of the claims 1-12, which is a continuous cyclic process and wherein each cycle comprises:
a) Applying and maintaining the stimulus for a certain period of time, with which the material gives off heat that is conducted outside the device;
b) Removing the stimulus with which the material cools; and
c) Using the cooled material to absorb heat from inside the device to be cooled.

14. Use of the organic-inorganic hybrid material of hexagonal packing crystalline structure of general formula ABX₃ (I) as defined in any of claims 1 - 11 as caloric material for cooling devices.

15. A device with cooling capacity induced by an external stimulus, comprising:
(1) An organic-organic hybrid material of general formula ABX₃ (I) and hexagonal packing crystalline structure as defined in any of claims 1 - 11,
(2) means to cyclically exercise the stimulus during a certain period of time on the organic-inorganic hybrid material and then remove it, where the external stimulus is selected from the group consisting of hydrostatic pressure, uniaxial pressure, electric field and illumination with light.

16. The device according to claim 15, wherein the organic-inorganic hybrid material is in powder form, suspended in a fluid, suspended in a solid matrix, or in the form of a thin film.
